# EUROPÄISCHE PATENTSCHRIFT

(11) **EP 0 445 643 B1**
(45) Veröffentlichungstag und Bekanntmachung des Hinweises auf die Patenterteilung: **17.05.1995**
(21) Anmeldenummer: 91102908.0
(22) Anmeldetag: 28.02.1991
(51) Int. Cl.: C07D 209/08

(54) **Verfahren zur Herstellung von 3,3-Dialkyl-1-methyl-2-methylenindolinen**
Process for preparation of 3,3-dialkyl-1-methyl-2-methyleneindolines
Procédé pour la préparation des 3,3-dialkyl-1-méthyle-2-méthylèneindolines

(30) Priorität: 06.03.1990 DE 4006920
(43) Veröffentlichungstag der Anmeldung: 11.09.1991
(73) Patentinhaber: BASF Aktiengesellschaft, 67063 Ludwigshafen (DE)
(72) Erfinder: Ebel, Klaus, Dr., D-6704 Mutterstadt (DE); Schroeder, Juergen, Dr., W-6806 Viernheim (DE)

(56) Entgegenhaltungen:
- DE-A- 2 154 246
- SYNTHESIS Mai 1986, STUTTGART Seiten 382 - 383; MANFRED LISSEL ET AL.: 'Dimethylcarbonat als Methylierungsmittel unter phasentransfer-katalytischen Bedingungen'

## Beschreibung

Die Erfindung betrifft ein neues Verfahren zur Herstellung von 3,3-Dialkyl-1-methyl-2-methylenindolinen durch Umsetzung von 3,3-Dialkyl-2-methylindoleninen mit Dimethylcarbonat.

Es sind aus Beilstein, Handbuch der organischen Chemie, Band 20, Hauptwerk Seite 324 bis 325 (1935) und 4. Ergänzungswerk Seite 3254 (1978) verschiedene Verfahren zur Herstellung von 1,3,3-Trialkyl-2-methylenindolinen bekannt. Für eine billige, technische Synthese sind diese Verfahren wegen ihrer aufwendigen Arbeitsweise und ihrer schlechten Ausbeuten ungeeignet.

Aus Synthesis 382 bis 383 (1986) ist ein Verfahren zur Alkylierung von heterocyclischen Verbindungen mit Dimethylcarbonat unter Phasen-Transfer-Katalyse bekannt, jedoch sind dort keine 2-Methylenindoline erwähnt.

Aus der DE-A-21 54 246 ist ein Verfahren zur Herstellung von 1,3,3-Trialkyl-2-methylenindolinen aus 3,3-Dialkyl-2-methylindoleninen mit Dialkylsulfat oder Alkylestern organischer Sulfonsäuren in Gegenwart alkalisch wirkender Verbindungen bekannt. Nachteilig bei diesem Verfahren ist der hohe Salzanfall, die hohe Toxizität der Dialkylsulfate und der hohe Preis der Alkylsulfonate.

Der vorliegenden Erfindung lag daher die Aufgabe zugrunde den zuvorgenannten Nachteilen abzuhelfen.

Demgemäß wurde ein neues und verbessertes Verfahren zur Herstellung von 3,3-Dialkyl-1-methyl-2-methylenindolinen der allgemeinen Formel I 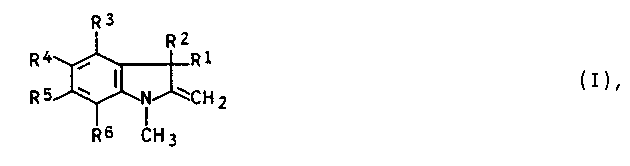
in der
- R¹, R²: unabhängig voneinander C₁- bis C₈-Alkyl
- R³, R⁴, R⁵, R⁶: unabhängig voneinander Wasserstoff C₁- bis C₄-Alkyl C₁- bis C₄-Halogenalkyl und Halogen bedeuten,
gefunden, welches dadurch gekennzeichnet ist, daß man 3,3-Dialkyl-2-methylindolenine der allgemeinen Formel II 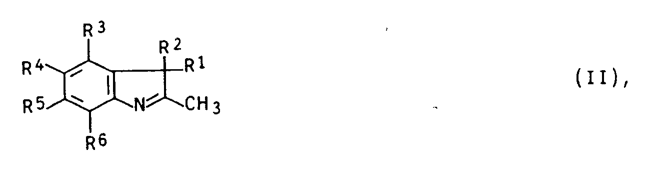
in der die Substituenten R¹ bis R⁶ die obengenannten Bedeutungen haben, mit Dimethylcarbonat bei Temperaturen von 150 bis 350°C und Drücken von 1 bis 200 bar umsetzt.

Das erfindungsgemäße Verfahren läßt sich in der Weise durchführen, daß man die 3,3-Dialkyl-2-methylindolenine der Formel II mit Dimethylcarbonat in stöchometrischer Menge, im über- oder Unterschuß, vorzugsweise im Molverhältnis von 0,01:1 bis 1:1, bevorzugt 0,1:1 bis 1:1, besonders bevorzugt 0,1:1 bis 0,5:1 3,3-Dialkyl-2-methylindolenin zu Dimethylcarbonat, umsetzt. Die Umsetzung läßt sich bei einer Temperatur oberhalb 150°C, im allgemeinen zwischen 150 und 350°C, vorzugsweise von 160 und 300°C, insbesondere von 170 und 270°C, drucklos oder unter Druck, vorzugsweise bei 1 bar bis 200 bar, besonders bevorzugt 1 bis 100 bar, zweckmäßig unter dem im Autoklaven bei vorgenannten Temperaturen sich einstellenden Dampfdruck des Reaktionsgemischs, kontinuierlich oder diskontinuierlich durchführen. Zweckmäßig dient das Reaktionsgemisch gleichzeitig als Lösungsmedium.

Im Vergleich zu den bekannten Verfahren liefert das Verfahren nach der Erfindung auf einfacherem und wirtschaftlicherem Wege 3,3-Dialkyl-1-methyl-2-methylenindoline in guter Ausbeute und Reinheit. Umständliche Neutralisationsoperationen sowie Korrosionsprobleme werden vermieden. Außer dem Zielprodukt werden lediglich ein Mol Methanol sowie das nicht toxische Kohlendioxid erhalten. Das erfindungsgemäße Verfahren ist somit umweltfreundlicher als die bekannten Verfahren. Der freiwerdende Methanol kann in bekannter Weise ohne Phosgen wieder mit Kohlenmonoxid und Sauerstoff zu Dimethylcarbonat verarbeitet werden (DE-A-23 34 736). Alle diese vorteilhaften Eigenschaften des erfindungsgemäßen Verfahrens sind im Hinblick auf den Stand der Technik überraschend.

Die Substituenten R¹ bis R⁶ in den Formeln I und II haben folgende Bedeutungen:
- R¹, R²: unbhängig voneinander
- C₁- bis C₈-Alkyl, vorzugsweise sind R¹ und R² gleich und bedeuten C₁- bis C₄-Alkyl wie Methyl, Ethyl, n-Propyl, iso-Propyl, n-Butyl, iso-Butyl, sec.-Butyl und tert.-Butyl,
- R³, R⁴, R⁵, R⁶: unabhängig voneinander
- Wasserstoff,
- C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl,
- C₁- bis C₄-Halogenalkyl, bevorzugt C₁- bis C₂-Fluor oder Chloralkyl wie Fluormethyl, Difluormethyl, Trifluormethyl, Chlormethyl, Dichlormethyl und Trichlormethyl, bevorzugt Trifluormethyl,
- Halogen wie Fluor, Chlor, Brom und Jod, bevorzugt Chlor und Brom,

3,3-Dialkyl-1-methyl-2-methylenindoline sind wertvolle Zwischenprodukte zur Herstellung von Methin- und Azomethinfarbstoffen (Ullmanns Encyklopädie der technischen Chemie, 4. Auflage, Band 16, Seite 635 bis 669), die in großem Umfang zum Färben von Polyacrylnitrilfasern Verwendung finden.

### Beispiele

Die in den folgenden Beispielen aufgeführten Teile bedeuten Gewichtsteile.

### Beispiel 1

15,9 Teile 2,3,3-Trimethylindolenin und 27,0 Teile Dimethylcarbonat werden in einem Autoklaven 9 Stunden auf 200°C erhitzt. Überschüssiges Dimethylcarbonat und Methanol werden bei Normaldruck abdestilliert. Man erhält 19,0 Teile Rückstand. Die gaschromatographische Analyse des Rückstandes zeigt, daß der Umsatz an 2,3,3-Trimethylindolenin 95 % beträgt. Die Ausbeute an 1,3,3-Trimethyl-2-methylenindolin beträgt 97 % (bezogen auf umgesetztes 2,3,3-Trimethylindolenin).

### Beispiel 2

19,4 Teile 5-Chlor-2,3,3-trimethylindolenin und 27,0 Teile Dimethylcarbonat werden in einem Autoklaven 15 Stunden auf 200°C erhitzt. Überschüssiges Dimethylcarbonat und Methanol werden bei Normaldruck abdestilliert. Man erhält 22,0 Teile Rückstand. Die gaschromatographische Analyse des Rückstandes zeigt, daß der Umsatz an 5-Chlor-2,3,3-trimethylindolenin 96 % beträgt. Die Ausbeute an 5-Chlor-1,3,3-trimethyl-2-methylenindolin beträgt 94 % (bezogen auf umgesetztes 5-Chlor-2,3,3-trimethylindolenin).

### Beispiel 3

Durch einen 2 m langen Rohrreaktor mit einem Durchmesser von 6 mm pumpt man bei 220°C und 50 bar stündlich 16,5 g einer Lösung aus 6,1 g 2,3,3-Trimethylindolenin und 10,4 g Dimethylcarbonat. Die gaschromatographische Analyse zeigt, daß der Umsatz an 2,3,3-Trimethylindolenin 94 % beträgt. Die Ausbeute an 1,3,3-Trimethyl-2-methylenindolin beträgt 99 % (bezogen auf umgesetztes 2,3,3-Trimethylindolenin).

## Patentansprüche

1. Verfahren zur Herstellung von 3,3-Dialkyl-1-methyl-2-methylenindolinen der allgemeinen Formel I 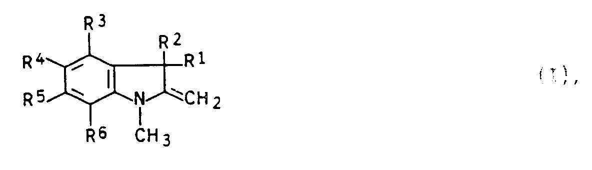 in der
R¹, R² unabhängig voneinander C₁- bis C₈-Alkyl,
R³, R⁴, R⁵, R⁶ unabhängig voneinander Wasserstoff, C₁- bis C₄-Alkyl, C₁- bis C₄-Halogenalkyl und Halogen bedeuten,
dadurch gekennzeichnet, daß man 3,3-Dialkyl-2-methylindolenine der allgemeinen Formel II 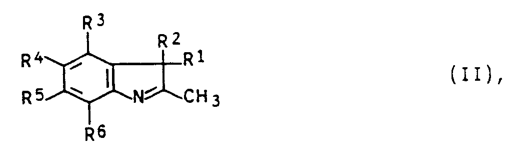 in der die Substituenten R¹ bis R⁶ die obengenannten Bedeutungen haben, mit Dimethylcarbonat bei Temperaturen von 150 bis 350°C und Drücken von 1 bis 200 bar umsetzt.

2. Verfahren nach Anspruch 1, dadurch gekennzeichnet, daß die Substituenten R¹ und R² gleich sind und C₁- bis C₄-Alkyl bedeuten.

3. Verfahren nach Anspruch 1, dadurch gekennzeichnet, daß man die Umsetzung bei Temperaturen von 160 bis 300°C durchführt.

4. Verfahren nach Anspruch 1, dadurch gekennzeichnet, daß man die Umsetzung bei Temperaturen von 170 bis 270°C durchführt.

5. Verfahren nach Anspruch 1, dadurch gekennzeichnet, daß man die Umsetzung bei Drücken von 1 bis 100 bar durchführt.

## Claims

1. A process for preparing 3,3-dialkyl-1-methyl-2-methyleneindolines of the general formula I 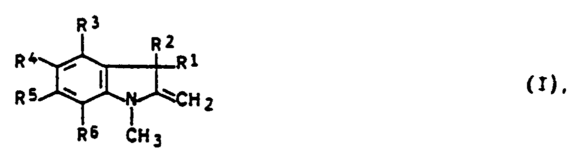 where
R¹ and R² are independently of each other C₁-C₈-alkyl, and
R³, R⁴, R⁵ and R⁶ are independently of one another hydrogen, C₁-C₄-alkyl, C₁-C₄-haloalkyl or halogen,
characterized in that 3,3-dialkyl-2-methylindolines of the general formula II 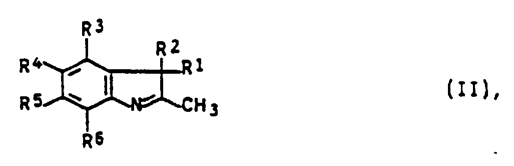 where the substituents R¹ to R⁶ are each as defined above, are reacted with dimethyl carbonate at temperatures from 150 to 350°C and pressures from 1 to 200 bar.

2. A process as claimed in claim 1, characterized in that the substituents R¹ and R² are identical and each is C₁-C₄-alkyl.

3. A process as claimed in claim 1, characterized in that the reaction is carried out at temperatures from 160 to 300°C.

4. A process as claimed in claim 1, characterized in that the reaction is carried out at temperatures from 170 to 270°C.

5. A process as claimed in claim 1, characterized in that the reaction is carried out at pressures from 1 to 100 bar.

## Revendications

1. Procédé de préparation de 3,3-dialkyl-1-méthyl-2-méthylèneindolines de formule générale I 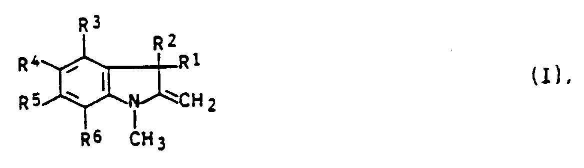 dans laquelle
R¹, R² représentent, indépendamment l'un de l'autre, des radicaux alkyle en C₁-C₈,
R³, R⁴, R⁵, R⁶ représentent, indépendamment les uns des autres, des atomes d'hydrogène, des radicaux alkyle en C₁-C₄, alkyle en C₁-C₄ halogénés ou des atomes d'halogène,
caractérisé en ce qu'on fait reagir des 3,3-dialkyl-2-méthylindolénines de formule générale II 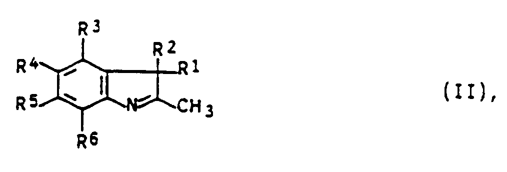 dans laquelle les substituants R¹ à R⁶ ont les significations indiquées ci-dessus, avec du carbonate de diméthyle à des températures de 150 à 350°C et sous des pressions de 1 a 200 bar.

2. Procedé selon la revendication 1, caractérisé en ce que les substituants R¹ et R² sont identiques et représentent des radicaux alkyle en C₁-C₄.

3. Procédé selon la revendication 1, caractérisé en ce qu'on conduit la réaction à des températures de 160 à 300°C.

4. Procédé selon la revendication 1, caractérisé en ce qu'on conduit la reaction à des températures de 170 a 270°C.

5. Procédé selon la revendication 1, caractérisé en ce qu'on conduit la reaction sous des Dressions de 1 à 100 bar.
